# EUROPÄISCHE PATENTSCHRIFT

(11) **EP 1 156 831 B1**
(45) Veröffentlichungstag und Bekanntmachung des Hinweises auf die Patenterteilung: **07.12.2005**
(21) Anmeldenummer: 00909212.3
(22) Anmeldetag: 18.02.2000
(51) Int. Cl.: A61K 47/36

(54) **HYDROXYETHYLSTÄRKE ZUR SCHMERZFREIEN UND GEWEBESCHONENDEN INJEKTION VON ARZNEIMITTELN**
HYDROXYETHY STARCH FOR THE PAINLESS AND TISSUE-CONSERVING INJECTION OF MEDICAMENTS
HYDROXYETHYL AMIDON POUR L'INJECTION DE MEDICAMENTS SANS DOULEUR ET EPARGNANT LES TISSUS

(30) Priorität: 21.02.1999 DE 19907257
(43) Veröffentlichungstag der Anmeldung: 28.11.2001
(73) Patentinhaber: B. BRAUN MELSUNGEN AG, 34212 Melsungen (DE)
(72) Erfinder: MEIER, Bernd, H., D-64285 Darmstadt (DE); JANKOWIAK-MEIER, Iris, D-64285 Darmstadt (DE)
(74) Vertreter: Weber, Thomas
(86) Internationale Anmeldenummer: PCT/EP2000/001357
(87) Internationale Veröffentlichungsnummer: WO 2000/048637

(56) Entgegenhaltungen:
- EP-A- 0 193 917
- FR-A- 2 296 429
- DATABASE WPI Section Ch, Week 199105 Derwent Publications Ltd., London, GB; Class B05, AN 1991-033716 XP002140935 & JP 02 304023 A (KYORIN), 17. Dezember 1990 (1990-12-17)

## Beschreibung

Die Erfindung betrifft eine wässrige Arzneimittellösung umfassend Hydroxyethylstärke mit einem Substitutionsgrad DS < 0,4 und wenigstens einen arzneilich wirksamen Bestandteil zur Infusion oder Injektion in Gefäße des menschlichen oder tierischen Körpers, ein Kit umfassend die Einzelbestandteile sowie injizierbare Fertigarzneimittel.

Die Injektion von Arzneimitteln in Blutgefäße des Körpers ist eine häufig angewendete Darreichungsform für Arzneistoffe. In der Regel wird eine bestimmte Menge des Arzneistoffs in einer wässrigen Lösung oder als Emulsion in eine unter der Haut verlaufende kleine Vene injiziert. Bei einer Vielzahl von Arzneistoffen treten jedoch unmittelbar nach der Injektion im Bereich des Gefäßes, in das der Arzneistoff injiziert wurde, Gewebsschäden und/oder Schmerzen auf. Als Ursache kommen zunächst die höheren Konzentrationen der schädigenden Arzneistoffe im Bereich der Injektionsstelle in Betracht. Darüber hinaus wird häufig die Injektion der Arzneistoffe mit einem hohen hydrostatischen Druck betrieben. Die Schäden werden dabei durch Kontakt des Gewebes der Gefäßwand und des umliegenden Bindegewebes mit den in höherer Konzentration schädigenden Arzneistoffen ausgelöst. Sehr häufig werden in wässrigen Lösungen vorliegende Arzneistoffe aus Stabilitätsgründen auf unphysiologische pH-Werte eingestellt. Nach Injektion wird durch Diffusion der Protonen oder Hydroxidionen (in Abhängigkeit von der Titrationsazidität der eingestellten Pufferlösungen) das Gewebe der Gefäßwand und des umliegenden Bindegewebes geschädigt. So ist beispielsweise bekannt, dass die versehentliche intra-arterielle Injektion des Narkosemittels Thiopental zu massiven Nekrosen im Verlauf des arteriellen Gefäßes bis hin zum Verlust der Extremität führen kann.

Nach dem bisherigen Stand der Technik wird diesen Schäden durch verschiedene Verfahren und Mittel entgegengewirkt. Am häufigsten versucht man Schäden durch Verdünnung der Injektionsarzneistoffe, entweder durch Einbringen in eine laufende Infusion, oder durch Mischen mit einer Infusionslösung vor der Infusion, zu verhindern. Allen Verdünnungsverfahren ist gemeinsam, dass eine verhältnismäßig kleine Menge des Arzneistoffes mit einem größeren Volumen einer Infusionslösung gemischt wird. Die Nachteile dieser Verdünnungsverfahren liegen in dem größeren Flüssigkeitsvolumen mit dem z. B. kreislaufkranke Patienten belastet werden sowie der sich daraus ergebenden Zeit für die Applikation, die für viele Arzneimittelwirkstoffe zu lang ist. Häufig wird ein Bolus, d. h. die Injektion eines kleinen Volumens mit konzentriertem Arzneistoff in kurzer Zeit, d. h., in wenigen Minuten oder Sekunden gewünscht.

Durch Infusion in Gefäßkatheter, die in größeren Gefäßen liegen (z. B. Zentralvenenkatheter), können potentiell schädigende Arzneistoffe nach Injektion durch das Patientenblut verdünnt werden. Hierbei ist die Anlage eines zentralen Venenkatheters mit allen Risiken (Fehlpunktion, bakterielle Besiedelung des Katheters und komplizierende Bakterieämie) und Kosten Bedingung. Ein weiteres Verfahren besteht darin, Arzneistoffe nicht in wässrigen Lösungen, sondern als Emulsion mit Fetten zu infundieren. Jedoch kommt es auch hier nach Injektion der Arzneistoffe zu Schäden und Schmerzen infolge des Übertritts der Arzneistoffe in die Gefäßwand und das Bindegewebe.

Kolloidosmotisch wirksame Makromoleküle wie Hydroxyethylstärke, Dextrane und andere Polysaccharide oder Polypeptide wie Polygelatine oder Albumin wurden bislang als Bestandteil von Blut- und Plasmaersatzlösungen (Volumenersatzmittel, Plasmaexpander) eingesetzt. Sie dienen dabei als Ersatz der kolloidosmotischen Wirkung der körpereigenen Plasmaeiweißstoffe und werden deshalb in haemodynamisch relevanten Mengen, also mehrere Gramm pro Tag, infundiert. Das Infusionsvolumen dieser haemodynamisch wirksamen Lösungen liegt in einem Bereich von mehreren hundert Millilitern.

So beschreibt die US-A-5,434,191 ein künstliches Blut in Form einer wässrigen Emulsion umfassend Wasser, einen Emulgator, ein synthetisches Phospholipid, eine perfluorierte Verbindung als Sauerstoffträger sowie eine Verbindung die ausgewählt ist aus der Gruppe bestehend aus Hydroxyethylstärke, Polyvinylpyrolidon, modifizierte Gelatine, Dextran oder ein ähnliche Substanz, welche für einen kolloidosmotischen Druck sorgt. Wegen der mit künstlichem Blut verbundenen Nierenkomplikationen hat sich dieses System als Blutersatz bislang jedoch noch nicht durchgesetzt.

EP-A-0650736 beschreibt die Verwendung von rekombinantem Humanserum-Albumin (rHSA) zur Erhöhung der Blutplasmamenge, zur Ergänzung des zirkulierenden Blutvolumens, zur Verbesserung der Hypoproteinemia und zur Erhaltung des kolloidosmotischen Druckes.

Wie bei Elektrolytlösungen, z. B. physiologischer Kochsalzlösungen, können und dürfen auch einige kolloidale Plasmaersatzlösungen als Trägerlösung für Medikamente verwendet werden. Hierzu wird ein Medikament der Infusionslösung während der Infusion zugespritzt und damit in mehreren hundert Millilitern der Lösung verdünnt (Rote Liste 1997, Editio Cantor Verlag, Nr. 52275, Haemaccel). Prinzipiell wird durch Infusion dieser Substanzen eine andauernde Steigerung des kolloidosmotischen Drucks im Blut bewirkt. Um diesen Effekt für einen hinreichend langen Zeitraum zu gewährleisten, weisen alle hemodynamisch wirksamen Plasmaersatzstoffe eine Halbwertszeit im Bereich bis zu einigen Stunden auf. Alle diese länger im Blut verweilenden kolloidosmotisch wirksamen Makromoleküle werden dabei zunächst von Zellen des retikuloendothelialen Systems aufgenommen und je nach Substanz gespeichert.
In besonderer Anwendung werden Kolloide zur Expansion des interstitiellen Raumes genutzt. So beschreibt die US-A-5,424,288 ein Verfahren zur Behandlung von Tumorkrebs in Lebewesen durch Verabreichung einer Suspension von makroaggregiertem Albumin in einem inerten Fluid in den Tumor, gefolgt von der Injektion von einem kolloidalen radioaktivem Agens.

V. Bocci beschreibt im Journal of Biological Response Modifiers (1985) 4/4 (340 - 352) ein Verfahren, um den interstitiellen Bindegewebsraum für subcutan oder intramuskulär applizierte Arzneistoffe zu vergrößern, die dann in die Lymphwege gelangen. FR-A-2296429 beschreibt ein Verfahren zur Verbesserung der Toleranz der intravenösen Injektion von Gammaglobulinen durch Zugabe von makromolekularen Substanzen, wie z.B. Hydroxyethylstärke, Dextranen und Albuminen.

WO-A-00/15766 offenbart eine pharmazeutische Zusammensetzung umfassend einen Wirkstoff, der den Golgi-Apparat beeinträchtigt in Kombination mit einem biokompatiblen Träger, wie z.B. einem Polysaccharid.

Die Aufgabe der Erfindung ist es, Arzneimittel in Gefäße des menschlichen und tierischen Körpers durch geeignete Maßnahmen so verabreichbar zu machen, dass Gefäßschädigungen, insbesondere lokale Gefäßschädigungen, des perivaskulären Bindegewebes stark reduziert werden und im Idealfall ganz unterbleiben und/oder dass die mit der Verabreichung verbundenen Schmerzen nur in verminderter Form auftreten und im Idealfall ganz unterbleiben. Durch geeignete Maßnahmen soll also diesen von der Arzneimittelkonzentration abhängigen Nebenwirkungen auf das Injektionsgefäß entgegengewirkt werden. Hierbei versteht man unter Injektionsgefäß dasjenige Gefäß (Vene, Arterie, etc.), in welches die Arzneimittelzusammensetzung verabreicht wird und welches mit anderen Gefäßen in Verbindung steht. Ferner war es eine Aufgabe der vorliegenden Erfindung, eine injizierbare wässrige Arzneimittellösung (Arzneimittel), insbesondere als Fertigarzneimittel oder als Kit zur Verfügung zu stellen.

Eine erste Ausführungsform der Erfindung betrifft injizierbare wässrige Arzneimittellösung umfassend wenigstens einen arzneilich wirksamen Bestandteil und Hydroxyethylstärke [Poly(O-Hydroxyethyl)stärke] mit einen Substitutionsgrad DS < 0,4.

Eine weitere Ausführungsform der Erfindung betrifft ein Kit umfassend getrennt voneinander (a) in wässriger Lösung Hydroxyethylstärke [Poly(O-Hydroxyethyl)stärke] mit einem Substitutionsgrad DS < 0,4 und (b) einen Arzneiwirkstoff.

Eine dritte Ausführungsform der Erfindung betrifft ein injizierbares Fertigarrzneimittel umfassend die injizierbare wässrige Arzneimittellösung wie oben erwähnt.

Die Arzneimittellösung ist dazu besonders geeignet arzneilich wirksame Bestandteile schmerzfrei und/oder gewebeschonend in Gefäße des menschlichen und tierischen Körpers einzubringen.

Oft sind die Gefäßschädigungen mit schmerzverursachenden Irritationen des perivaskulären Bindegewebes verbunden, so dass gemäß der Erfindung Gefäßschädigungen, insbesondere lokale Gefäßschädigungen, sowie schmerzverufsachende Irritationen des perivaskulären Bindegewebes reduziert und im Idealfall ganz vermieden werden können.

Das erfindungsgemäße Prinzip beugt der Vaskulitis vor, d.h. einer entzündlichen Reaktion, die von der Wand der Gefäße, insbesondere der Blutgefäße des menschlichen und tierischen Körpers ihren Ausgang nimmt.

Gemäß der Erfindung wird die Diffusion des arzneilich wirksamen Bestandteils durch die Gefäßwände des Injektionsgefäßes und die damit verbundenen lokalen Gefäßschädigungen sowie die gegebenenfalls zusätzlich auftretenden schmerzverursachenden Irritationen des perivaskulären Bindegewebes deutlich vermindert, vorzugsweise ganz verhindert.

Erfindungsgemäß handelt es sich bei den kolloidosmotisch wirksamen Substanzen um natürliche oder synthetische Kolloide bildende Makromoleküle, welche in wässriger Lösung einen kolloidosmotischen Druck von > 1333 Pa (10 mmHg), vorzugsweise > 3733 Pa (28 mmHg, entsprechend dem KOD von Plasma), aufweisen.

Gemäß der Erfindung kommen solche Hydroxyethylstärken in Betracht, welche einen Substitutionsgrad (Degree of Substitution) DS < 0,4 aufweisen. Der Substitutionsgrad DS ist definiert als der Anteil der substituierten Anhydroglucose-Einheiten aller Anhydroglucose-Einheiten. Ihn kann man bestimmen aus der gemessenen Menge der unsubstituierten Glucose nach Hydrolyse einer Probe. Vorzugsweise beträgt der Substitutionsgrad DS wenigstens 0,10, besonders bevorzugt wenigstens 0,15.

Die erfindungsgemäß eingesetzten Hydroxyethylstärken weisen vorzugsweise ein mittleres Molekulargewicht von unter 300.000, vorzugsweise von unter 70.000 und ganz besonders bevorzugt von unter 40.000 auf. Besonders bevorzugt besitzt die Hydroxyethylstärke einen Substitutionsgrad DS zwischen 0,1 und < 0,4 und ein mittleres Molekulargewicht von unter 300.000.

Im Gegensatz zu Kolloiden, die als Plasmaexpander eingesetzt werden, ist gemäß der Erfindung eine anhaltende Wirkung auf den kolloidosmotischen Druck im Blut, bzw. ein längeres Verbleiben im Blut als zusätzliche pharmakologische Wirkung nicht erwünscht. Für den erfindungsgemäß beabsichtigten Effekt ist lediglich ein Verbleiben in der Strombahn des Injektionsgefäßes und ein kurzfristig wirkender kolloidosmotischer Effekt notwendig. Idealerweise werden die Substanzen schon bei der ersten Nierenpassage wieder aus dem Körper ausgeschieden. Es ist bekannt, dass die Serumhalbwertszeit der Hydroxyethylstärke in erster Linie von ihrem Substitutionsgrad DS abhängt. Das Molekulargewicht spielt hierbei eine eher untergeordnete Rolle. Für die Wasserlöslichkeit der Stärke ist jedoch eine Mindestmenge von Hydroxyethylgruppen notwendig. Eine niedrig substituierte Hydroxyethylstärke mit einem Substitutionsgrad unter 0,4, zum Beispiel mit einer mittleren Substitution von 0,3, wäre bei hinreichend großen Molekulargewichten im Infusionsgefäß befriedigend kolloidosmotisch wirksam, würde jedoch nach Infusion sehr schnell wieder aus dem Körper ausgeschieden (z. B. renal eliminiert). Diese Hydroxyethylstärke wäre als Plasmaexpander wegen der raschen Elimination wenig geeignet. Eine relevante Belastung des Retikuloendothelialen Systems bzw. eine kumulierende Organspeicherung würde auch nach wiederholter Injektion nicht stattfinden.
Bei den anderen oben genannten kolloidalen Plasmaexpandern besteht ein pharmakokinetischer Zusammenhang zwischen Molekulargewicht und Serumhalbwertszeit. Entsprechend ist bei Dextranen mit einem mittleren Molekulargewicht unter 40.000, vorzugsweise unter 20.000 eine entsprechend schnellere Elimination aus dem Körper zu beobachten. Auch bei Oxypolygelatine bzw. Gelatinesuccinat kann durch ein mittleres Molekulargewicht kleiner als 40.000, vorzugsweise kleiner als 20.000, besonders bevorzugt kleiner als 15.000, eine schnelle Elimination aus dem Körper bewirkt werden. Auch durch quantitativ größere Infusionen dieser Lösungen mit kleineren mittleren Molekulargewichten kann dauernd keine wesentliche Steigerung des kolloidosmotischen Drucks im Patientenblut ausgelöst werden.
Die Anwendung eines kolloidosmotisch wirksamen Prinzips bedeutet unabhängig von der Pharmakokinetik eine Injektion von geringen Mengen kolloidaler Makromoleküle. Ein Volumen von 10 ml einer beanspruchten Injektionslösung mit einer 10%igen Kolloidkonzentration beinhaltet 1g Kolloid. Wogegen das Zuspritzen von Arzneimitteln in kolloidale Plasmaersatzlösungen (Plasmaexpander) als Trägerlösungen eine Kolloidbelastung von 50g, eine signifikante Erhöhung des kolloidosmotischen Drucks im Blut des Patienten und eine häufig unerwünschte Verdünnung bzw. zu langsame Verabreichung des Arzneistoffes nach sich zieht.

Der Anteil der kolloidosmotisch wirksamen Substanz beträgt, bezogen auf die Gesamtmenge der injizierbaren wässrigen Lösung, 2 bis 25 Gew.%, vorzugsweise 3 bis 15 Gew.-%.

Die erfindungsgemäß zur Anwendung kommenden injizierbaren wässrigen Lösungen eines Arzneimittels weisen eine Osmolarität zwischen 250 und 400 mOsmol/l, vorzugsweise zwischen 300 und 350 mOsmol/l auf. Die Einstellung der Tonizität der zu injizierenden Lösung kann durch einen zusätzlichen Kationenanteil von 100 - 170 mmol/l, vorzugsweise 100 - 150 mmol/l, und einen Anionenanteil von 100 bis 140 mmol/l, vorzugsweise 100 - 150 mmol/l vorgenommen werden. Ein Anteil der Kationen- und/oder Anionenkonzentration kann durch ein natürliches oder synthetisches Polyol ersetzt sein. Zu diesem Zweck geeignete Polyole sind im Stand der Technik bekannt. Beispielhaft seien in diesem Zusammenhang Zucker wie Glucose und synthetische Polyole wie Sorbitol oder Xylitol genannt.

Der pH-Wert der zur Anwendung kommenden Lösung ist im wesentlichen bestimmt von den an die Stabilität des zu verabreichenden Arzneimittels in der wässrigen Lösung geknüpften Erfordernissen. Der pH-Wert kann zwischen 1,5 und 12, vorzugsweise zwischen 4,5 und 8, liegen. Die erfindungsgemäß verwendeten Lösungen sind partikelfrei und frei von emulsionsbildenden perfluorierten organischen Verbindungen. Bei den erfindungsgemäß verwendeten Zusammensetzungen handelt es sich also weder um Emulsionen noch um Suspensionen. Die injizierbaren Zusammensetzungen können ferner in der infusionstherapie übliche und physiologisch verträgliche Zusatzstoffe und Hilfsstoffe enthalten, wie beispielsweise Puffersysteme. Als Puffer kann der Lösung die Aminosäure Histidin, vorzugsweise in einer Dosierung von 50 - 150 mmol/l und Histidinhydrochlorid, vorzugsweise in einer Dosierung von 5 - 20 mmol/l zugesetzt werden.

Erfindungsgemäß können als arzneilich wirksamer Bestandteil Arzneimittel eingesetzt werden, die ausgewählt sind aus der Gruppe bestehend aus Abmagerungsmittel/Appetitzügler, Acidosetherapeutika, Aminosäuren (z. B. Histidin) oder modifizierte Aminosäuren, Analeptika/Antihypoxämika, Analgetika/Antirheumatika, Anthelminthika, Antiallergika, Antianämika, Antiarrhythmika, Antibiotika/Antiinfektiva, Antidementiva (Nootropika), Antidiabetika, Antidota, Antiemetika/Antivertiginosa, Antiepileptika, Antihämorrhagika (Antifibrinolytika u. andere Hämostatika), Antihypertonika, Antihypoglykämika, Antihypotonika, Antikoagulantia, Antimykotika, Antiparasitäre Mittel (intern), Antiphlogistika, Antitussiva/Expektorantia, Arteriosklerosemittel, Balneotherapeutika und Mittel zur Wärmetherapie, Betarezeptoren-, Calciumkanalblocker und Hemmstoffe des Renin-Angiotensin-Systems, Broncholytika/Antiasthmatika, Cholagoga und Gallenwegstherapeutika, Cholinergika, Corticoide (intern), Dermatika (intern), Diätetika/Ernährungstherapeutika, Diagnostika und Mittel zur Diagnosevorbereitung, Diuretika, Durchblutungsfördernde Mittel, Entwöhnungsmittel, Enzyminhibitoren, Enzympräparate und Transportproteine, Fibrinolytika, Geriatrika, Gichtmittel, Grippemittel, Gynäkologika, Hämorrhoidenmittel (Proktologika), Hepatika, Hypnotika/Sedativa, Hypophysen-, Hypothalamushormone, regulatorische Peptide und ihre Hemmstoffe, Immuntherapeutika und Zytokine, Infusions- und Standardinjektionslösungen, Organperfusionslösungen, Kardiaka, Karies-, Parodontosemittel und andere Dentalpräparate, Koronarmittel, Laxantia, Lipidsenker, Neuraltherapeutika, Magen-Darm-Mittel, Migränemittel, Mineralstoffpräparate, Muskelrelaxantia, Narkosemittel, Nebenschildddrüsenhormone/Calciumstoffwechselregulatoren/Osteoporosemittel, Neuropathiepräparate und andere neurotrope Mittel, Neurotransmitter (z. B. Dopamin) oder modifizierte Neurotransmitter, Ophthalmika, Otologika, Parkinsonmittel und andere Mittel gegen extrapyramidale Störungen, Psychopharmaka, Sinusitismittel, Roborantia/Tonika, Schilddrüsentherapeutika, Sera, Immunglobuline und Impfstoffe, Sexualhormone und ihre Hemmstoffe, Spasmolytika, Thrombozytenaggregationshemmer, Tuberkulosemittel, Umstimmungsmittel, Urologika, Venentherapeutika, Vitamine, Wundbehandlungsmittel, Zytostatika und Metastasenhemmer. Der Arzneimittelwirkstoff kann in der injizierbaren Lösung in einem Anteil von 0,5 von 25 Gew.-% vorzugsweise von 2 bis 15 Gew.%, besonders bevorzugt 5 bis 10 Gew.% enthalten sein, bezogen auf die Gesamtmenge der injizierbaren Lösung.

Die erfindungsgemäß verwendeten wässrigen Arzneimittellösungen können durch die verwendeten Kolloide im Hinblick auf ihre Wasserlöslichkeit, Stabilität, Fließeigenschaften und Viskosität günstig beeinflusst werden. Desgleichen sind vorteilhafte Veränderungen hinsichtlich der elektrischen Leitfähigkeit, der Filtrationseigenschaften, der Temperaturleitfähigkeit, der akustischen Resonanz, der Chemolumineszenz sowie Phagocytierbarkeit durch entsprechende Auswahl der kolloidosmotisch wirksamen Substanz möglich.

Die Erfindung betrifft ferner einen Kit umfassend die erfindungsgemäß verwendeten Zusammensetzungsbestandteile in getrennter Form. Im einzelnen umfasst das Kit die kolloidosmotisch wirksame Substanz in wässriger Lösung sowie getrennt davon den Arzneiwirkstoff in fester, flüssiger oder gelöster Form. Hinsichtlich der kolloidosmotisch wirksamen Substanz und den Arzneiwirkstoffen wird auf die obigen Ausführungen verwiesen. Die beiden Komponenten werden vor der Applikation (Injektion, Infusion) miteinander gemischt und mit Hilfe eines Perfusors und/oder einer Infusionsmaschine, die ebenfalls unabhängig voneinander in dem Kit enthalten sein können, verabreicht. Der Perfusor bzw. die Infusionsmaschine kann durch einen Prozessor gesteuert werden, der/die von einem Meßgerät oder Eingabegerät Signale empfängt. Hinsichtlich der Eigenschaften und der übrigen Bestandteile, die in der wässrigen injizierbaren Lösung enthalten sein können, wird auf die obigen Ausführungen verwiesen.

In einer weiteren besonderen Ausgestaltung betrifft die Erfindung eine injizierbare wässrige Arzneimittellösung umfassend wenigstens einen arzneilich wirksamen Bestandteil, der aus der oben beschriebenen Gruppe ausgewählt ist, und Hydroxyethylstärke mit einem Substitutionsgrad DS < 0,4 als kolloidosmotisch wirksame Substanz

In Form ihres Fertigarzneimittels sind die oben beschriebenen injizierbaren wässrigen Arzneimittellösungen dazu geeignet, Arzneimittel zu injizieren, die, in anderer Form injiziert, zu Gewebeschädigungen und/oder Schmerzen führen würden.

Die Herstellung der injizierbaren wässrigen Arzneimittellösung kann nach bekannten Methoden des Standes der Technik erfolgen, insbesondere durch Mischen der Einzelkomponenten unter Bildung der Lösung. Zur Herstellung des Kits werden die Einzelkomponenten in geeignete Behältnisse gefüllt, verschlossen und getrennt voneinander in Form des Kits bis zur Anwendung zur Verfügung gestellt. Gemäß der Erfindung können Arzneimittelwirkstoffe schnell und in hohen Konzentrationen injiziert werden, wobei die Gefäßschädigungen, insbesondere lokale Gefäßschädigungen am Injektionsort vermindert sind, in der Regel sogar ganz unterbleiben. Bedingt durch die Reduktion der Irritationen des perivaskulären Bindegewebes treten kaum oder keine Schmerzen an der Injektionsstelle auf.

Besonders vorteilhaft ist die Möglichkeit zur Verabreichung kleiner Boli, insbesondere von Injektionsboli von bis zu 20 ml, vorzugsweise bis zu 10 ml, besonders bevorzugt bis zu 5 ml oder Infusionsboli von bis zu 100 ml, vorzugsweise bis zu 50 ml.

### Beispiel

20 mg Etomidat wurden in einer 10 Gew.-%igen wässrigen Lösung von Hydroxyethylstärke (DS < 0,4; mittleres Molekulargewicht < 70.000) in Gegenwart von 100 mmol/l Anionenanteil und 100 mmol/l Kationenanteil gelöst und intravenös rasch injiziert. Die Injektion verlief schmerzfrei.

## Patentansprüche

1. Kit umfassend getrennt voneinander (a) in wässriger Lösung Hydroxyethylstärke [Poly(O-Hydroxyethyl)stärke] mit einem Substitutionsgrad DS < 0,4 und (b) einen Arzneiwirkstoff.

2. Kit gemäß Anspruch 1, **dadurch gekennzeichnet, dass** der Arzneiwirkstoff in fester, flüssiger oder gelöster Form vorliegt.

3. Kit gemäß irgendeinem der vorherigen Ansprüche, **dadurch gekennzeichnet, dass** die Hydroxyethylstärke ein mittleres Molekulargewicht von unter 300.000 aufweist.

4. Kit gemäß irgendeinem der vorherigen Ansprüche, **dadurch gekennzeichnet, dass** der Arzneiwirkstoff ausgewählt ist aus der Gruppe bestehend aus Abmagerungsmittel/Appetitzügler, Acidosetherapeutika, Aminosäuren und modifizierte Aminosäuren, Analeptika/Antihypoxämika, Analgetika/Antirheumatika, Anthelminthika, Antiallergika, Antianämika, Antiarrhythmika, Antibiotika/Antünfektiva, Antidementiva (Nootropika), Antidiabetika, Antidota, Antiemetika/Antivertiginosa, Antiepileptika, Antihämorrhagika (Antifibrinolytika und andere Hämostatika), Antihypertonika, Antihypoglykämika, Antihypotonika, Antikoagulantia, Antimykotika, Antiparasitäre Mittel, Antiphlogistika, Antitussiva/Expektorantia, Arteriosklerosemittel, Balneotherapeutika und Mittel zur Wärmetherapie, Betarezeptoren-, Calciumkanalblocker und Hemmstoffe des Renin-Angiotensin-Systems, Broncholytika/Antiasthmatika, Cholagoga und Gallenwegstherapeutika, Cholinergika, Corticoide (intern), Dermatika (intern), Diätetika/ Ernährungstherapeutika, Diagnostika und Mittel zur Diagnosevorbereitung, Diuretika, Durchblutungsfördernde Mittel, Entwöhnungsmittel, Enzyminhibitoren, Enzympräparate und Transportproteine, Fibrinolytika, Geriatrika, Gichtmittel, Grippemittel, Gynäkologika, Hämorrhoidenmittel (Proktologika), Hepatika, Hypnotika/Sedativa, Hypophysen-, Hypothalamushormone, regulatorische Peptide und ihre Hemmstoffe, Immuntherapeutika und Zytokine, Infusions- und Standardinjektionslösungen, Organperfusionslösungen, Kardiaka, Karies-, Parodontosemittel und andere Dentalpräparate, Koronarmittel, Laxantia, Lipidsenker, Neuraltherapeutika, Magen-Darm-Mittel, Migränemittel, Mineralstoffpräparate, Muskelrelaxantia, Narkosemittel, Nebenschilddrüsenhormone /Calciumstoffwechselregulatoren/Osteoporosemittel, Neuropathiepräparate und andere neurotrope Mittel, Neurotransmitter und modifizierte Neurotransmitter, Ophthalmika, Otologika, Parkinsonmittel und andere Mittel gegen extrapyramidale Störungen, Psychopharmaka, Sinusitismittel, Roborantia/Tonika, Schilddrüsentherapeutika, Sera, immunglobuline und impfstoffe, Sexualhormone und ihre Hemmstoffe, Spasmolytika, Thrombozytenaggregationshemmer, Tuberkulosemittel, Umstimmungsmittel, Urologika, Venentherapeutika, Vitamine, Wundbehandlungsmittel, Zytostatika und Metastasenhemmer.

5. Injizierbare wässrige Arzneimittellösung umfassend wenigstens einen arzneilich wirksamen Bestandteil und Hydroxyethylstärke [Poly(O-Hydroxyethyl)stärke] mit einen Substitutionsgrad DS < 0,4.

6. Injizierbare wässrige Arzneimittellösung gemäß Anspruch 5, **dadurch gekennzeichnet, dass** die Hydroxyethylstärke ein mittleres Molekulargewicht von unter 300.000 aufweist.

7. Injizierbare wässrige Arzneimittellösung gemäß irgendeinem der vorigen Ansprüche, **dadurch gekennzeichnet, dass** die Hydroxyethylstärke in wässriger Lösung einen kolloidosmotischen Druck von > 3733 Pa (28 mmHg) aufweist.

8. Injizierbare wässrige Arzneimittellösung gemäß irgendeinem der vorigen Ansprüche, **dadurch gekennzeichnet, dass** der Anteil der Hydroxyethylstärke, bezogen auf die Gesamtmenge der injizierbaren wässrigen Lösung, 2 bis 25 Gew.-% beträgt.

9. Injizierbare wässrige Arzneimittellösung gemäß irgendeinem der vorigen Ansprüche, **dadurch gekennzeichnet, dass** der Anteil der Hydroxyethylstärke, bezogen auf die Gesamtmenge der injizierbaren wässrigen Lösung 3 bis 15 Gew.- % beträgt.

10. Injizierbare wässrige Arzneimittellösung gemäß irgendeinem der vorigen Ansprüche, **dadurch gekennzeichnet, dass** der arzneilich wirksame Bestandteil, bezogen auf die Gesamtmenge der injizierbaren wässrigen Lösung, in einem Anteil von 0,5 bis 25 Gew.-% vorliegt.

11. Injizierbare wässrige Arzneimittellösung gemäß irgendeinem der vorigen Ansprüche, **dadurch gekennzeichnet, dass** der arzneilich wirksame Bestandteil ausgewählt ist aus der Gruppe bestehend aus Abmagerungsmittel/Appetitzügler, Acidosetherapeutika, Aminosäuren und modifizierte Aminosäuren, Analeptika/Antihypoxämika, Analgetika/Antirheumatika, Anthelminthika, Antiallergika, Antianämika, Antiarrhythmika, Antibiotika/Antiinfektiva, Antidementiva (Nootropika), Antidiabetika, Antidota, Antiemetika/Antivertiginosa, Antiepileptika, Antihämorrhagika (Antifibrinolytika und andere Hämostatika), Antihypertonika, Antihypoglykämika, Antihypotonika, Antikoagulantia, Antimykotika, Antiparasitäre Mittel, Antiphlogistika, Antitussiva/Expektorantia, Arteriosklerosemittel, Balneotherapeutika und Mittel zur Wärmetherapie, Betarezeptoren-, Calciumkanalblocker und Hemmstoffe des Renin-Angiotensin-Systems, Broncholytika/Antiasthmatika, Cholagoga und Gallenwegstherapeutika, Cholinergika, Corticoide (intern), Dermatika (intern),DiätetikalErnährungstherapeutika, Diagnostika und Mittel zur Diagnosevorbereitung, Diuretika, Durchblutungsfördernde Mittel, Entwöhnungsmittel, Enzyminhibitoren, Enzympräparate und Transportproteine, Fibrinolytika, Geriatrika, Gichtmittel, Grippemittel, Gynäkologika, Hämorrhoidenmittel (Proktologika), Hepatika, Hypnotika/Sedativa, Hpophysen-, Hypothalamushormone, regulatorische Peptide und ihre Hemmstoffe, Immuntherapeutika und Zytokine, Infusions- und Standardinjektionslösungen, Organperfusionslösungen, Kardiaka, Karies-, Parodontosemittel und andere Dentalpräparate, Koronarmittel, Laxantia, Lipidsenker, Neuraltherapeutika, Magen-Darm-Mittel, Migränemittel, Mineralstoffpräparate, Muskelrelaxantia, Narkosemittel, Nebenschildddrüsenhormone/Calciumstoffwechselregulatoren/Osteoporosemittel, Neuropathiepräparate und andere neurotrope Mittel, Neurotransmitter und modifizierte Neurotransmitter, Ophthalmika, Otologika, Parkinsonmittel und andere Mittel gegen extrapyramidale Störungen, Psychopharmaka, Sinusitismittel, Roborantia/Tonika, Schilddrüsentherapeutika, Sera, Immunglobuline und Impfstoffe, Sexualhormone und ihre Hemmstoffe, Spasmolytika, Thrombozytenaggregationshemmer, Tuberkulosemittel, Umstimmungsmittel, Urologika, Venentherapeutika, Vitamine, Wundbehandlungsmittel, Zytostatika und Metastasenhemmer.

12. Injizierbares Fertigarzneimittel umfassend die injizierbare wässrige Arzneimittellösung gemäß irgendeinem der Ansprüche 5 bis 11.

## Claims

1. A kit comprising separately (a) hydroxyethylstarch [poly(O-hydroxyethyl)-starch] having a degree of substitution, DS, of < 0.4 in an aqueous solution and (b) a pharmaceutically active ingredient.

2. The kit according to claim 1, **characterized in that** said pharmaceutically active ingredient is in a solid, liquid or dissolved form.

3. The kit according to any of the preceding claims, **characterized in that** said hydroxyethylstarch has an average molecular weight of below 300,000.

4. The kit according to any of the preceding claims, **characterized in that** said pharmaceutically active ingredient is selected from the group consisting of slimming preparations/anorexiants, acidose therapeutics, amino acids and modified amino acids, analeptics/antihypoxemics, analgetics/antirheumatics, anthelmintics, antiallergics, antianemics, antiarrhythmics, antibiotics/antiinfectives, antidementives (nootropics), antidiabetics, antidotes, antiemetics/antivertiginosics, antiepileptics, antihemorrhagics (antifibrinolytics and other hemostatics), antihypertensives, antihypoglycemics, antihypotensives, anticoagulants, antimycotics, antiparasitics, antiphlogistics, antitussives/expectorants, anti-arteriosclerosis agents, balneotherapeutics and agents for heat therapy, beta receptor and calcium channel blockers and inhibitors of the renin-angiotensin system, broncholytics/antiasthmatics, cholagogics and bile duct therapeutics, cholinergics, corticoids (internal), dermatics (internal), dietetics/nutrition therapeutics, diagnostics and agents for diagnostic preliminaries, diuretics, agents stimulating blood flow, withdrawal agents, enzyme inhibitors, enzyme preparations and transport proteins, fibrinolytics, geriatric agents, gout agents, influenza remedies, gynecologic agents, anti-hemorrhoidal agents (proctologics), hepatics, hypnotics/sedatives, hypophysis and hypothalamus hormones, regulatory peptides and their inhibitors, immunotherapeutics and cytokines, infusion and standard injection solutions, organ perfusion solutions, cardiacs, anti-caries and anti-parodontosis agents and other dental preparations, coronary agents, laxants, lipid depressants, neural therapeutics, gastro-intestinal agents, migraine remedies, mineral preparations, muscle relaxants, narcotics, parathyroid hormones/calcium-metabolic regulators/osteoporosis remedies, neuropathy preparations and other neurotropic agents, neurotransmitters and modified neurotransmitters, ophthalmics, otologics, Parkinson remedies and other remedies against extrapyramidal disturbances, psychopharmacons, sinusitis remedies, roborants/tonics, thyroid therapeutics, serums, immunoglobulins and vaccines, sexual hormones and their inhibitors, spasmolytics, platelet aggregation inhibitors, tuberculosis remedies, alterants, urologic agents, vein therapeutics, vitamins, wound treatment agents, cytostatics and metastasis inhibitors.

5. An injectable aqueous medicinal solution comprising at least one pharmaceutically active ingredient and hydroxyethylstarch [poly(O-hydroxyethyl)-starch] having a degree of substitution, DS, of < 0.4.

6. The injectable aqueous medicinal solution according to claim 5, **characterized in that** said hydroxyethylstarch has an average molecular weight of below 300,000.

7. The injectable aqueous medicinal solution according to any of the preceding claims, **characterized in that** said hydroxyethylstarch has a colloid-osmotic pressure in aqueous solution of > 3733 Pa (28 mm Hg).

8. The injectable aqueous medicinal solution according to any of the preceding claims, **characterized in that** the proportion of hydroxyethylstarch is from 2 to 25% by weight, based on the total amount of the injectable aqueous solution.

9. The injectable aqueous medicinal solution according to any of the preceding claims, **characterized in that** the proportion of hydroxyethylstarch is from 3 to 15% by weight, based on the total amount of the injectable aqueous solution.

10. The injectable aqueous medicinal solution according to any of the preceding claims, **characterized in that** said pharmaceutically active ingredient is contained in a proportion of from 0.5 to 25% by weight, based on the total amount of the injectable aqueous solution.

11. The injectable aqueous medicinal solution according to any of the preceding claims, **characterized in that** said pharmaceutically active ingredient is selected from the group consisting of slimming preparations/anorexiants, acidose therapeutics, amino acids and modified amino acids, analeptics/ anti-hypoxemics, analgetics/antirheumatics, anthelmintics, antiallergics, antianemics, antiarrhythmics, antibiotics/antiinfectives, antidementives (nootropics), antidiabetics, antidotes, antiemetics/antivertiginosics, antiepileptics, antihemorrhagics (antifibrinolytics and other hemostatics), antihypertensives, antihypoglycemics, antihypotensives, anticoagulants, antimycotics, antiparasitics, antiphlogistics, antitussives/expectorants, anti-arteriosclerosis agents, balneotherapeutics and agents for heat therapy, beta receptor and calcium channel blockers and inhibitors of the renin-angiotensin system, broncholytics/antiasthmatics, cholagogics and bile duct therapeutics, cholinergics, corticoids (internal), dermatics (internal), dietetics/nutrition therapeutics, diagnostics and agents for diagnostic preliminaries, diuretics, agents stimulating blood flow, withdrawal agents, enzyme inhibitors, enzyme preparations and transport proteins, fibrinolytics, geriatric agents, gout agents, influenza remedies, gynecologic agents, anti-hemorrhoidal agents (proctologics), hepatics, hypnotics/sedatives, hypophysis and hypothalamus hormones, regulatory peptides and their inhibitors, immunotherapeutics and cytokines, infusion and standard injection solutions, organ perfusion solutions, cardiacs, anti-caries and anti-parodontosis agents and other dental preparations, coronary agents, laxants, lipid depressants, neural therapeutics, gastro-intestinal agents, migraine remedies, mineral preparations, muscle relaxants, narcotics, parathyroid hormones/calcium-metabolic regulators/osteoporosis remedies, neuropathy preparations and other neurotropic agents, neurotransmitters and modified neurotransmitters, ophthalmics, otologics, Parkinson remedies and other remedies against extrapyramidal disturbances, psychopharmacons, sinusitis remedies, roborants/tonics, thyroid therapeutics, serums, immunoglobulins and vaccines, sexual hormones and their inhibitors, spasmolytics, platelet aggregation inhibitors, tuberculosis remedies, alterants, urologic agents, vein therapeutics, vitamins, wound treatment agents, cytostatics and metastasis inhibitors.

12. An injectable ready medicament comprising the injectable aqueous medicinal solution according to any of claims 5 to 11.

## Revendications

1. Kit comprenant, séparés l'un de l'autre, (a) en solution aqueuse de l'hydroxyéthylamidon [poly(O-hydroxyéthyl)amidon] ayant un degré de substitution DS < 0,4 et (b) un principe actif médicamenteux.

2. Kit selon la revendication 1 **caractérisé en ce que** le principe actif médicamenteux est sous forme solide, liquide ou dissoute.

3. Kit selon l'une quelconque des revendications précédentes **caractérisé en ce que** l'hydroxyéthylamidon présente une masse moléculaire moyenne inférieure à 300000.

4. Kit selon l'une quelconque des revendications précédentes **caractérisé en ce que** le principe actif médicamenteux est choisi dans le groupe consistant en les agents amaigrissants/freinant l'appétit, les agents thérapeutiques contre l'acidose, les aminoacides et aminoacides modifiés, les analeptiques/antihypoxémiques, les analgésiques/antirhumatismaux, les antihelmintiques, les antiallergiques, les antianémiques, les antiarythmiques, les antibiotiques/anti-infectieux, les agents antidémence (nootropes), les antidiabétiques, les antidotes, les antiémétiques/antivertigineux, les antiépileptiques, les antihémorragiques (antifibrinolytiques et autres hémostatiques), les antihypertoniques, les antihypoglycémiques, les antihypotoniques, les anticoagulants, les antimycotiques, les agents antiparasitaires, les antiphlogistiques, les antitussifs/expectorants, les agents contre l'artériosclérose, les agents balnéothérapeutiques et les agents pour la thermothérapie, les inhibiteurs des bêta-récepteurs, .les inhibiteurs calciques et les inhibiteurs du système rénine-angiotensine, les broncholytiques/antiasthmatiques, les cholagogues et les agents thérapeutiques pour les voies biliaires, les cholinergiques, les corticoïdes (internes), les agents dermiques (internes), les agents diététiques/agents thérapeutiques pour l'alimentation, les agents diagnostiques et les agents pour la préparation d'un diagnostic, les diurétiques, les agents favorisant la circulation sanguine, les agents de sevrage, les inhibiteurs d'enzymes, les préparations enzymatiques et les protéines de transport, les fibrinolytiques, les agents gériatriques, les agents contre la goutte, les agents contre la grippe, les agents gynécologiques, les agents contre les hémorroïdes (proctologiques), les agents hépatiques, le hypnotiques/sédatifs, les hormones de l'hypophyse, de l'hypothalamus, les peptides régulateurs et leurs inhibiteurs, les agents immunothérapeutiques et les cytokines, les solutions pour perfusions et injections standard, les solutions pour perfusions d'organes, les agents cardiaques, les agents contre les caries, la parodontose et autres préparations dentaires, les agents coronariens, les laxatifs, les agents abaissant les lipides, les agents thérapeutiques neuraux, les agents pour l'estomac et l'intestin, les agents contre la migraine, les préparations de substances minérales, les relaxants musculaires, les narcotiques, les hormones de la thyroïde accessoire/régulateurs du métabolisme du calcium/agents contre l'ostéoporose, les préparations contre les neuropathies et autres agents neurotropes, les neurotransmetteurs et les neurotransmetteurs modifiés, les agents ophtalmiques, les agents otologiques, les agents contre la maladie de Parkinson et autres agents contre les troubles extrapyramidaux, le agents psychopharmaceutiques, les agents contre la sinusite, les agents roboratifs/toniques, les agents thérapeutiques pour la thyroïde, les sérums, les immunoglobulines et les vaccins, les hormones sexuelles et leurs inhibiteurs, les spasmolytiques; les inhibiteurs de l'agrégation des thrombocytes, les agents contre la tuberculose, les agents contre les changements d'humeur, les agents urologiques, les agents véinothérapeutiques, les vitamines, les agents de traitement des plaies, les cytostatiques et les inhibiteurs de métastases.

5. Solution médicamenteuse aqueuse injectable comprenant au moins un constituant efficace du point de vue médicamenteux et de l'hydroxyéthylamidon [poly(O-hydroxyéthyl)amidon] ayant un degré de substitution DS < 0,4.

6. Solution médicamenteuse aqueuse injectable selon la revendication 5 **caractérisée en ce que** l'hydroxyéthylamidon présente une masse moléculaire moyenne inférieure à 300000.

7. Solution médicamenteuse aqueuse injectable selon l'une quelconque des revendications précédentes **caractérisée en ce que** l'hydroxyéthylamidon présente en solution aqueuse une pression colloïdoosmotique > 3733 Pa (28 mm Hg).

8. Solution médicamenteuse aqueuse injectable selon l'une quelconque des revendications précédentes **caractérisée en ce que** la proportion d'hydroxyéthylamidon, par rapport à la quantité totale de solution aqueuse injectable, est 2 à 25 % en masse.

9. Solution médicamenteuse aqueuse injectable selon l'une quelconque des revendications précédentes **caractérisée en ce que** la proportion d'hydroxyéthylamidon, par rapport à la quantité totale de solution aqueuse injectable, est 3 à 15 % en masse.

10. Solution médicamenteuse aqueuse injectable selon l'une quelconque des revendications précédentes **caractérisée en ce que** le constituant efficace du point de vue médicamenteux, par rapport à la quantité totale de solution aqueuse injectable, est présent en une proportion de 0,5 à 25 % en masse.

11. Solution médicamenteuse aqueuse injectable selon l'une quelconque des revendications précédentes **caractérisée en ce que** le constituant efficace du point de vue médicamenteux est choisi dans le groupe consistant en les agents amaigrissants/freinant l'appétit, les agents thérapeutiques contre l'acidose, les aminoacides et aminoacides modifiés, les analeptiques/antihypoxémiques, les analgésiques/antirhumatismaux, les antihelmintiques, les antiallergiques, les antianémiques, les antiarythmiques, les antibiotiques/anti-infectieux, les agents antidémence (nootropes), les antidiabétiques, les antidotes, les antiémétiques/antivertigineux, les antiépileptiques, les antihémorragiques (antifibrinolytiques et autres hémostatiques), les antihypertoniques, les antihypoglycémiques, les antihypotoniques, les anticoagulants, les antimycotiques, les agents antiparasitaires, les antiphlogistiques, les antitussifs/expectorants, les agents contre l'artériosclérose, les agents balnéothérapeutiques et les agents pour la thermothérapie, les inhibiteurs des bêta-récepteurs, les inhibiteurs calciques et les inhibiteurs du système rénine-angiotensine, les broncholytiques/antiasthmatiques, les cholagogues et les agents thérapeutiques pour les voies biliaires, les cholinergiques, les corticoïdes (internes), les agents dermiques (internes), les agents diététiques/agents thérapeutiques pour l'alimentation, les agents diagnostiques et les agents pour la préparation d'un diagnostic, les diurétiques, les agents favorisant la circulation sanguine, les agents de sevrage, les inhibiteurs d'enzymes, les préparations enzymatiques et les protéines de transport, les fibrinolytiques, les agents gériatriques, les agents contre la goutte, les agents contre la grippe, les agents gynécologiques, les agents contre les hémorroïdes (proctologiques), les agents hépatiques, le hypnotiques/sédatifs, les hormones de l'hypophyse, de l'hypothalamus, les peptides régulateurs et leurs inhibiteurs, les agents immunothérapeutiques et les cytokines, les solutions pour perfusions et injections standard, les solutions pour perfusions d'organes, les agents cardiaques, les agents contre les caries, la parodontose et autres préparations dentaires, les agents coronariens, les laxatifs, les agents abaissant les lipides, les agents thérapeutiques neuraux, les agents pour l'estomac et l'intestin, les agents contre la migraine, les préparations de substances minérales, les relaxants musculaires, les narcotiques, les hormones de la thyroïde accessoire/régulateurs du métabolisme du calcium/agents contre l'ostéoporose, les préparations contre les neuropathies et autres agents neurotropes, les neurotransmetteurs et les neurotransmetteurs modifiés, les agents ophtalmiques, les agents otologiques, les agents contre la maladie de Parkinson et autres agents contre les troubles extrapyramidaux, le agents psychopharmaceutiques, les agents contre la sinusite, les agents roboratifs/toniques, les agents thérapeutiques pour la thyroïde, les sérums, les immunoglobulines et les vaccins, les hormones sexuelles et leurs inhibiteurs, les spasmolytiques, les inhibiteurs de l'agrégation des thrombocytes, les agents contre la tuberculose, les agents contre les changements d'humeur, les agents urologiques, les agents véinothérapeutiques, les vitamines, les agents de traitement des plaies, les cytostatiques et les inhibiteurs de métastases.

12. Médicament fini injectable comprenant la solution médicamenteuse aqueuse injectable selon l'une quelconque des revendications 5 à 11.
